# EUROPEAN PATENT SPECIFICATION

(11) **EP 3 397 285 B1**
(45) Date of publication and mention of the grant of the patent: **14.08.2024**
(21) Application number: 17723020.8
(22) Date of filing: 21.04.2017
(51) Int. Cl.: A61K 9/20, A61K 31/4545, A61K 31/47

(54) **TABLET FORMULATIONS OF MONTELUKAST SODIUM AND RUPATADINE FUMARATE**
TABLETTENFORMULIERUNGEN VON MONTELUKAST-NATRIUM UND RUPATADIN-FUMARAT
FORMULATIONS DE MONTÉLUKAST SODIQUE ET DE FUMARATE DE RUPATADINE EN COMPRIMÉS

(30) Priority: 22.04.2016 TR 201605261; 14.12.2016 TR 201618525
(43) Date of publication of application: 07.11.2018
(73) Proprietor: Sanovel Ilac Sanayi ve Ticaret A.S., 34460 Istanbul (TR)
(72) Inventor: TÜRKYILMAZ, Ali, 34460 Istanbul (TR); AYHAN, Merve, 34460 Istanbul (TR); PEHLIVAN AKALIN, Nur, 34460 Istanbul (TR); DEMIR, Isil, 34460 Istanbul (TR)
(74) Representative: Sevinç, Erkan
(86) International application number: PCT/EP2017/059541
(87) International publication number: WO 2017/182644

(56) References cited:
- WO-A1-2015/069203
- NIDHI NK ET AL: "ANALYTICAL METHOD DEVELOPMENT AND VALIDATION OF SIMULTANEOUS ESTIMATION OF RUPATADINE FUMARATE AND MONTELUKAST SODIUM BY RP-HPLC", 1 January 2014 (2014-01-01), pages 393 - 399, XP055380575, Retrieved from the Internet <URL:http://www.ijpra.com/File_Folder/393-399(ijpra).pdf>
- VIVEKKUMAR K. REDASANI ET AL: "Stability indicating RP-HPLC method for simultaneous estimation of rupatadine fumarate and montelukast sodium in bulk and tablet dosage form", JOURNAL OF ANALYTICAL CHEMISTRY, vol. 69, no. 4, 26 March 2014 (2014-03-26), pages 384 - 389, XP055169763, ISSN: 1061-9348, DOI: 10.1134/S1061934814040121
- VASANTH PM ET AL: "INTERNATIONAL RESEARCH JOURNAL OF PHARMACY DEVELOPMENT AND SIMULTANEOUS VALIDATION OF STABILITY INDICATING RP-HPLC METHOD FOR THE ASSAY OF MONTELUKAST AND RUPATADINE IN SOLID DOSAGE FORM INTRODUCTION Montelukast sodium is chemically 2", 1 January 2012 (2012-01-01), XP055380883, Retrieved from the Internet <URL:http://www.irjponline.com/admin/php/uploads/1539_pdf.pdf>

## Description

### Field of Invention

The present invention relates to a monolayer tablet formulation comprising montelukast sodium in combination with rupatadine fumarate.

### Background of Invention

Montelukast sodium has one asymmetric center and is the R-isomer. A crystalline and an amorphous form are known. Montelukast sodium is a leukotriene receptor antagonist approved for the prophylaxis and chronic treatment of asthma. The chemical name of montelukast is [R-(E)]-1-[[[1-[3-[2-(7-chloro-2-quinolinyl)ethenyl]phenyl]-3-[2-(1-hydroxy-1-methylethyl)-phenyl]propyl]thio]methyl] cyclopropane acetic acid mono sodium salt and has the structure shown in the following formula I. It works by blocking the action of substances in the body that cause the symptoms of asthma and allergic rhinitis. It is used to prevent wheezing, difficulty breathing, chest tightness, and coughing caused by asthma.

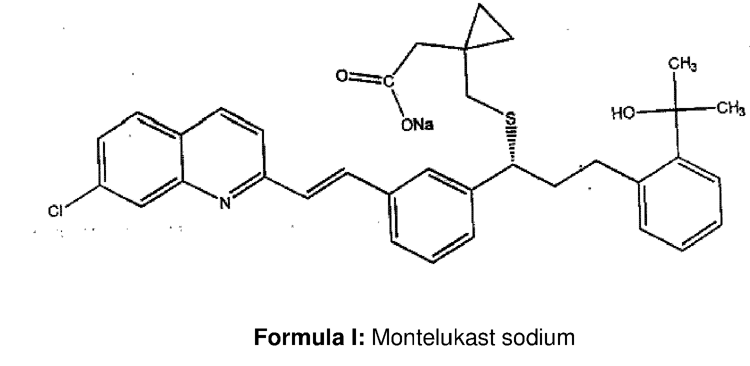

In patent WO9716173, tablet formulation of montelukast sodium has been disclosed. It also exists in the market as tablet, chewable tablet and oral granule under the brand name of SINGULAIR^{®} in U.S.

Rupatadine is a second generation antihistamine and PAF (Platelet-activating factor) antagonist used to treat allergies and is marketed under several trade names such as Rupafin, Alergoliber, Rinialer, Pafinur, Rupax and Ralif.

Rupatadine fumarate has been approved for the treatment of allergic rhinitis and chronic urticaria in adults and children over 12 years. The defined daily dose (DDD) is 10 mg orally. The chemical name of rupatadine fumarate is 8-Chloro-6,11-dihydro-11-[1-[(5-methyl-3-pyridyl)methyl]-4-piperidylidene]-5H-benzo[5,6]cyclohepta[1,2-b]pyridine fumarate and has the structure shown in the following formula II.

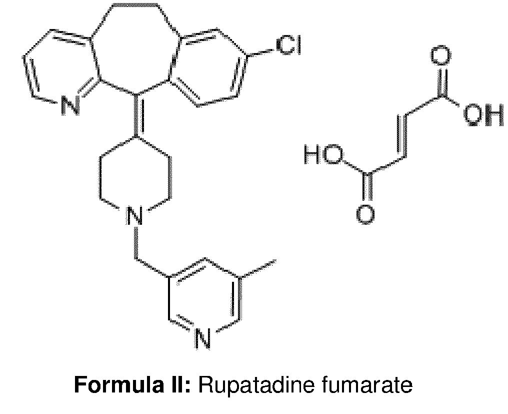

Rupatadine is first disclosed in US6803468 (Ranbaxy Laboratories Limited) and there are several patent applications describing, its process and other salt forms.

In prior art, there may have some capsule formulations including montelukast or rupatadine alone or may be in combination. However, a capsule formulation is less effective than a tablet combination and this may cause compatibility and stability problems and moreover dissolution problems of active drug substances.

It has also been found that the combination of rupatadine and montelukast is more effective compared to rupatadine alone in the control of allergic rhinitis symptoms.

In addition, instead of one per use, combining more than one molecule in one dosage form increases the patients quality of life and patients compliance. Considering all these, combinations of montelukast and rupatadine in a suitable pharmaceutical dosage formulation such as a tablet formulation is needed in the art. However, there are many challenges while combining two or more molecules in a tablet such as dissolution and stability problems.

In prior art, there are other pharmaceutical dosage forms comprising montelukast sodium and rupatadine fumarate but the process described in prior art are complex, time consuming and costs are high.

In this invention, to overcome the problems mentioned above, a monolayer tablet formulation of montelukast and rupatadine which is defined in claim 1, has been developed by using spray granulation method which is simple and cost-effective method.

The invention is as set out in the appended set of claims.

### Description of the invention

The main object of the present invention is to obtain a monolayer tablet formulation comprising a stable and compatible combination of montelukast sodium and rupatadine fumarate with a desired dissolution and stability.

According to this embodiment, the present invention is aimed to obtain a stable combination formulation with synergistic effect for use in the treatment of asthma.

A further object of the present invention is to provide a simple, cost-effective, and time-saving process for preparing an improved monolayer tablet formulation of a combination of montelukast sodium and rupatadine fumarate, which is easily dissolved and therefore has high absorption and high bioavailability.

A further object of the present invention is to provide less side effects. It has been found that during the process of a monolayer tablet of montelukast sodium and rupatadine fumarate combination, less excipient has been used than other pharmaceutical dosage form of montelukast sodium and rupatadine fumarate combination in prior art. Using less excipients gives a monolayer tablet advantages such as having less weight and volume and having less side effects caused by excipients.

Another object of the present invention is to obtain selection of excipients in a certain ratio has more importance to provide high bioavailability and dissolution.

In order to combine two different molecules in one dosage form, molecules should be compatible with each other to achieve desired stability and dissolution for the patients compliance. During the development study to combine montelukast and rupatadine, it has been found that montelukast shows stability and dissolution problems when it is used together with rupatadine in a tablet formulation or in capsule form. The analysis results that, the combination in tablet or capsule form shows an increase in impurity and a decrease in dissolution of montelukast. In this invention, spray granulation method is used and desired dissolution and stability has been achieved. Dissolution results of the two active drug substances are given below in Example 2, in more detail.

According to this embodiment, a monolayer tablet formulation comprises montelukast sodium and rupatadine fumarate wherein the formulation comprises 2.00 to 10.00 % by weight of montelukast sodium as drug substance and 2.00 to 10.00 % by weight of rupatadine fumarate as drug substance and pregelatinized starch and croscarmellose sodium as disintegrants, wherein the total amount of pregelatinized starch in the composition is from 1.00 to 5.00 % by weight and the total amount of croscarmellose sodium in the composition is from 1.00 to 3.00 % by weight; wherein the weight ratio of croscarmellose sodium to pregelatinized starch is 0.05-5.0; wherein said monolayer tablet formulation further comprises
a. 1.00 to 3.00 % of hydroxypropyl cellulose
b. 40.00 to 55.00 % of microcrystalline cellulose
c. 40.00 to 55.00 % of lactose monohydrate
d. 0.10 to 2.00 % of magnesium stearate;
and wherein said formulation is obtained by means of a spray granulation method with applying purified water.

According to the invention, croscarmellose sodium is very effective excipient in the preparation of the tablet. It has been surprisingly found that the object of the present invention is achieved when pregelatinized starch and croscarmellose sodium are used in a certain ratio. Consequently, the ratio of croscarmellose sodium to pregelatinized starch is be in the range of 0.05-5.0 by weight. Said ratio provides adequate disintegrating properties for the tablets of the present invention but also stabilizes the active pharmaceutical ingredients which does not degrade to unwanted impurities.

According to this embodiment, the weight ratio of croscarmellose sodium to pregelatinized starch is 0.05-5.0.

According to the invention the binder is hydroxylpropyl cellulose.

According to the invention the fillers are microcrystalline cellulose and lactose monohydrate.

According to the invention, the lubricant is magnesium stearate.

According to this embodiment, formulation is obtained by means of spray granulation method with applying a purified water.

Another aspect of the present invention is to provide a process for preparing the monolayer tablet formulation comprising the following steps:
a) mixing montelukast sodium, rupatadine fumarate, hydroxypropyl cellulose (Klucel LF), half of the croscarmellose sodium, pregelatinized starch, microcrystalline cellulose Ph 101, lactose monohydrate for 15 minutes,
b) the mixture is added to fluidized bed dryers, spray granulation method is applied with purified water, then drying the mixture until maximum 2% humidity left,
c) adding the rest of croscarmellose sodium to step (b) mixture and further mixing for 15 minutes,
d) adding magnesium stearate to step(c) mixture and further mixing for 5 minutes,
e) compressing the mixture into tablets.

### Example 1

| **Agents** | **% by weight** |
|---|---|
| montelukast sodium | 2.00 - 10.00 |
| rupatadine fumarate | 2.00 - 10.0 |
| hydroxypropyl cellulose (Klucel LF) | 1.00 - 3.00 |
| microcrystalline cellulose (PH 101) | 40.00 - 55.00 |
| lactose monohydrate | 40.00 - 55.00 |
| croscarmellose sodium | 1.00 - 3.00 |
| pregelatinized starch | 1.00 -5.00 |
| magnesium stearate | 0.10 - 2.00 |
| pure water* | 35.00 - 45.00 |

| | |
|---|---|
| * It is used during granulation, then it is removed from the medium during the drying step. | |

### Process for example 1

Process for preparing the tablet formulation in example 1 comprises the following steps;
a) mixing montelukast sodium, rupatadine fumarate, hydroxypropyl cellulose (Klucel LF), half of the croscarmellose sodium, pregelatinized starch, microcrystalline cellulose pH 101, lactose monohydrate for 15 minutes,
b) the mixture is added to fluidized bed dryers, spray granulation method is applied with purified water, then drying the mixture until maximum 2% humidity left,
c) adding the rest of croscarmellose sodium to step (b) mixture and further mixing for 15 minutes,
d) adding magnesium stearate to step(c) mixture and further mixing for 5 minutes,
e) compressing the mixture into tablets.

### Example 2 (Dissolution profile test)

The tablet formulation of this present invention (Example 1), was tested by its dissolution profile in 0.5% solution of sodium dodecyl sulphate and at 37°C using an apparatus II method in 900 mL, rotating at 75 rpm. The results are shown below in Table 1.

**Table 1: Dissolution profile test results (%) at the end of 20 minutes**

| | **% Montelukast** | **% Rupatadin** |
|---|---|---|
| *Serial Nu:* | *First serial* | *First serial* |
| 1.Tablet | 94.7 | 97.6 |
| 2.Tablet | 96.1 | 99.5 |
| 3.Tablet | 103.9 | 101.5 |
| 4.Tablet | 100.1 | 102.8 |
| 5.Tablet | 102.0 | 100.6 |
| 6.Tablet | 93.2 | 97.1 |
| **Mean** | **98** | **100** |

Therefore it is shown that when these molecules are formulated together in one layer tablet form, the dissolution of montelukast and rupatadin is higher.

## Claims

1. A monolayer tablet formulation comprising montelukast sodium and rupatadine fumarate wherein the formulation comprises 2.00 to 10.00 % by weight of montelukast sodium as drug substance and 2.00 to 10.00 % by weight of rupatadine fumarate as drug substance and pregelatinized starch and croscarmellose sodium as disintegrants, wherein the total amount of pregelatinized starch in the composition is from 1.00 to 5.00 % by weight and the total amount of croscarmellose sodium in the composition is from 1.00 to 3.00 % by weight; wherein the weight ratio of croscarmellose sodium to pregelatinized starch is 0.05-5.0; wherein said monolayer tablet formulation further comprises
a. 1.00 to 3.00 % of hydroxypropyl cellulose
b. 40.00 to 55.00 % of microcrystalline cellulose
c. 40.00 to 55.00 % of lactose monohydrate
d. 0.10 to 2.00 % of magnesium stearate;
and wherein said formulation is obtained by means of a spray granulation method with applying purified water.

2. Process for preparing the monolayer tablet formulation according to claim 1, comprising the following steps;
a. mixing montelukast sodium, rupatadine fumarate, hydroxypropyl cellulose, half of the croscarmellose sodium, pregelatinized starch, microcrystalline cellulose, lactose monohydrate for 15 minutes,
b. the mixture is added to fluidized bed dryers and the spray granulation method is applied with purified water, then drying the mixture until maximum 2% humidity left,
c. adding the rest of croscarmellose sodium to step(b) mixture and further mixing for 15 minutes,
d. adding magnesium stearate to step(c) mixture and further mixing for 5 minutes,
e. compressing the mixture into tablets.

## Patentansprüche

1. Einschichtige Tablettenformulierung umfassend Montelukast-Natrium und Rupatadinfumarat, wobei die Formulierung 2,00 bis 10,00 Gew.% Montelukast-Natrium als Arzneimittelwirkstoff und 2,00 bis 10,00 Gew.% Rupatadinfumarat als Arzneimittelmittelwirkstoff und vorgelatinierte Stärke und Croscarmellose-Natrium als Sprengmittel umfasst, wobei die Gesamtmenge an vorgelatinierter Stärke in der Zusammensetzung 1.00 bis 5,00 Gew.% und die Gesamtmenge an Croscarmellose-Natrium in der Zusammensetzung 1,00 bis 3,00 Gew.% beträgt; wobei das Gewichtsverhältnis von Croscarmellose-Natrium zu vorgelatinierter Stärke 0,05-5,0 beträgt; wobei die einschichtige Tablettenformulierung weiterhin umfasst
a. 1,00 bis 3,00 % Hydroxypropylcellulose
b. 40,00 bis 55,00 % mikrokristalline Cellulose
c. 40,00 bis 55,00 % Laktosemonohydrat
d. 0,10 bis 2,00 % Magnesiumstearat;
und wobei die Formulierung durch ein Sprühgranulationsverfahren unter Verwendung von gereinigtem Wasser erhalten wird.

2. Verfahren zur Herstellung der einschichtigen Tablettenformulierung nach Anspruch 1,
umfassend die folgenden Schritte;
a. Mischen von Montelukast-Natrium, Rupatadinfumarat, Hydroxypropylcellulose, der Hälfte des Croscarmellose-Natriums, vorgelatinierter Stärke, mikrokristalliner Cellulose und Laktosemonohydrat für 15 Minuten,
b. die Mischung wird in einen Wirbelschichttrockner gegeben und das Sprühgranulationsverfahren mit gereinigtem Wasser angewandt, dann Trochnen der Mischung bis maximal 2% Feuchtigkeit verbleibend,
c. Zugabe des restlichen Croscarmellose-Natriums zur Mischung aus Schritt b) und weiteres Mischen für 15 Minuten,
d. Zugabe von Magnesiumstearat zur Mischung aus Schritt (c) und weiteres Mischen für 5 Minuten,
e. Pressen der Mischung zu Tabletten.

## Revendications

1. - Formulation de comprimé monocouche comprenant du montélukast sodique et du fumarate de rupatadine, la formulation comprenant de 2,00 à 10,00 % en poids de montélukast sodique en tant que substance médicamenteuse et de 2,00 à 10,00 % en poids de fumarate de rupatadine en tant que substance médicamenteuse et de l'amidon prégélatinisé et de la croscarmellose sodique en tant que désintégrants, la quantité totale d'amidon prégélatinisé dans la composition étant de 1,00 à 5,00 % en poids et la quantité totale de croscarmellose sodique dans la composition étant de 1,00 à 3,00 % en poids ; le rapport pondéral de la croscarmellose sodique à l'amidon prégélatinisé étant de 0,05 à 5,0 ; la formulation de comprimé monocouche comprenant en outre
a. 1,00 à 3,00 % d'hydroxypropyl cellulose
b. 40,00 à 55,00 % de cellulose microcristalline
c. 40,00 à 55,00 % de lactose monohydraté
d. 0,10 à 2,00 % de stéarate de magnésium ;
et ladite formulation étant obtenue au moyen d'un procédé de granulation par pulvérisation avec application d'eau purifiée.

2. - Procédé de préparation de la formulation de comprimé monocouche selon la revendication 1, comprenant les étapes suivantes ;
a. mélanger le montélukast sodique, le fumarate de rupatadine, l'hydroxypropyl cellulose, la moitié de la croscarmellose sodique, l'amidon prégélatinisé, la cellulose microcristalline, le lactose monohydraté pendant 15 minutes ;
b. le mélange est ajouté à des séchoirs à lit fluidisé et le procédé de granulation par pulvérisation est appliqué avec de l'eau purifiée, puis le mélange est séché jusqu'à 2 % maximum d'humidité restante ;
c. ajouter le reste de croscarmellose sodique au mélange de l'étape (b) et continuer à mélanger pendant 15 minutes ;
d. ajouter du stéarate de magnésium au mélange de l'étape (c) et continuer à mélanger pendant 5 minutes ;
e. comprimer le mélange en comprimés.
